# EUROPEAN PATENT SPECIFICATION

(11) **EP 2 066 327 B1**
(45) Date of publication and mention of the grant of the patent: **14.11.2012**
(21) Application number: 07803397.4
(22) Date of filing: 11.09.2007
(51) Int. Cl.: A61K 31/506, A61K 38/05, A61P 35/00, A61K 31/69, A61K 45/06

(54) **HISTONE DEACETYLASE INHIBITORS WITH COMBINED ACTIVITY ON CLASS-I AND CLASS-IIB HISTONE DEACETYLASES IN COMBINATION WITH PROTEASOME INHIBITORS**
HISTON-DEACETYLASE-HEMMER MIT KOMBINIERTER WIRKUNG GEGEN HISTON-DEACETYLASEN DER KLASSE I UND KLASSE IIB IN KOMBINATION MIT PROTEASOMHEMMERN
INHIBITEURS DE L'HISTONE DÉSACÉTYLASE À ACTIVITÉ COMBINÉE SUR LES HISTONE DÉSACÉTYLASES DE CLASSE I ET DE CLASSE IIB, COMBINÉS À DES INHIBITEURS DU PROTÉASOME

(30) Priority: 15.09.2006 EP 06120726; 03.05.2007 US 915895 P
(43) Date of publication of application: 10.06.2009
(73) Proprietor: Janssen Pharmaceutica NV, 2340 Beerse (BE)
(72) Inventor: ARTS, Janine, 2340 Beerse (BE); HELLEMANS, Peter Willem Jan, 2340 Beerse (BE); JANICOT, Michel Marie François, 2340 Beerse (BE); PAGE, Martin John, 2340 Beerse (BE)
(74) Representative: Vandersteen, Pieter
(86) International application number: PCT/EP2007/059518
(87) International publication number: WO 2008/031817

(56) References cited:
- WO-A-2006/010750
- HIDESHIMA T ET AL: "Small-molecule inhibition of proteasome and aggresome function induces synergistic antitumor activity in multiple myeloma" PROCEEDINGS OF THE NATIONAL ACADEMY OF SCIENCES OF USA, NATIONAL ACADEMY OF SCIENCE, WASHINGTON, DC, vol. 102, no. 24, 14 June 2005 (2005-06-14), pages 8567-8572, XP002407032 ISSN: 0027-8424
- LENTZSCH SUZANNE ET AL: "Combination of proteasome inhibitor PS 341 (Veleade (R)) with histone acetylase inhibitor (HDAC) PXD 101 shows superior anti-myeloma activity and inhibits osteoclastogenesis" BLOOD, W.B.SAUNDERS COMPANY, ORLANDO, FL, vol. 106, no. 11, pt. 1, 1 November 2005 (2005-11-01), page Abstr.2488, XP009096162 ISSN: 0006-4971
- MITSIADES C S ET AL: "Transcriptional signature of histone deacetylase inhibition in multiple myeloma: Biological and clinical implications" PROCEEDINGS OF THE NATIONAL ACADEMY OF SCIENCES OF USA, NATIONAL ACADEMY OF SCIENCE, WASHINGTON, DC, vol. 101, no. 2, 13 January 2004 (2004-01-13), pages 540-545, XP002407033 ISSN: 0027-8424
- PEI XIN-YAN ET AL: "Synergistic induction of oxidative injury and apoptosis in human multiple myeloma cells by the proteasome inhibitor bortezomib and histone deacetylase inhibitors" CLINICAL CANCER RESEARCH, THE AMERICAN ASSOCIATION FOR CANCER RESEARCH, vol. 10, no. 11, 1 June 2004 (2004-06-01), pages 3839-3852, XP002407048 ISSN: 1078-0432
- SUTHEESOPHON KRITTAYA ET AL: "Histone deacetylase inhibitor depsipeptide (FK228) induces apoptosis in leukemic cells by facilitating mitochondrial translocation of Bax, which is enhanced by the proteasome inhibitor bortezomib" ACTA HAEMATOLOGICA, S. KARGER, BASEL, CH, vol. 115, no. 1-2, 1 January 2006 (2006-01-01), pages 78-90, XP009096148 ISSN: 0001-5792
- TERZAH M HORTON ET AL: "Bortezomib interactions with chemotherapy agents in acute leukemia in vitro" CANCER CHEMOTHERAPY AND PHARMACOLOGY, SPRINGER-VERLAG, BE, vol. 58, no. 1, 1 July 2006 (2006-07-01), pages 13-23, XP019334374 ISSN: 1432-0843
- CATLEY LAURENCE ET AL: "Novel hydroxamic acid-derived HDAC inhibitor LBH589 potently activates intrinsic and extrinsic apoptotic pathways, and induces tubulin hyperacetylation in multiple myeloma" BLOOD, W.B.SAUNDERS COMPANY, ORLANDO, FL, vol. 106, no. 11, Part 1, 1 November 2005 (2005-11-01), page 453A, XP009096146 ISSN: 0006-4971
- YU CHUNRONG ET AL: "The proteasome inhibitor bortezomib interacts synergistically with histone deacetylase inhibitors to induce apoptosis in Bcr/Abl+ cells sensitive and resistant to STI571" BLOOD, W.B.SAUNDERS COMPANY, ORLANDO, FL, vol. 102, no. 10, 15 November 2003 (2003-11-15), pages 3765-3774, XP002471510 ISSN: 0006-4971
- BAI ET AL: "Histone deacetylase inhibitor trichostatin A and proteasome inhibitor PS-341 synergistically induce apoptosis in pancreatic cancer cells" BIOCHEMICAL AND BIOPHYSICAL RESEARCH COMMUNICATIONS, ACADEMIC PRESS INC. ORLANDO, FL, US, vol. 348, no. 4, 7 August 2006 (2006-08-07), pages 1245-1253, XP005614402 ISSN: 0006-291X
- PEI XINYAN ET AL: "The proteasome inhibitor Bortezomib interacts synergistically with histone deacetylase inhibitors to induce apoptosis in human multiple myeloma cells" BLOOD, W.B.SAUNDERS COMPANY, ORLANDO, FL, vol. 102, no. 11, 16 November 2003 (2003-11-16), page 685a, XP009096214 ISSN: 0006-4971
- GIMSING PETER ET AL: "Activity of the histone deacetylase (HDAC) inhibitor PXD101 in preclinical studies and in a phase I study in patients with advanced haematological tumors." BLOOD, vol. 106, no. 11, Part 1, November 2005 (2005-11), pages 932A-933A, XP008092865 & 47TH ANNUAL MEETING OF THE AMERICAN-SOCIETY-OF-HEMATOLOGY; ATLANTA, GA, USA; DECEMBER 10 -13, 2005 ISSN: 0006-4971
- BASU SUPRATIK ET AL: "The histone deacetylase inhibitor sodium valproate has in vitro anti-neoplastic activity against primary multiple myeloma cells and cell lines both alone and in combination with Velcade." BLOOD, vol. 106, no. 11, Part 2, November 2005 (2005-11), page 370B, XP008092867 & 47TH ANNUAL MEETING OF THE AMERICAN-SOCIETY-OF-HEMATOLOGY; ATLANTA, GA, USA; DECEMBER 10 -13, 2005 ISSN: 0006-4971
- HIDESHIMA TERU ET AL: "Small molecule inhibition of proteasome and aggresome function induces synergistic anti-tumor activity in multiple myeloma" BLOOD, W.B.SAUNDERS COMPANY, ORLANDO, FL, vol. 106, no. 11, pt. 1, 1 November 2005 (2005-11-01), page Abstr.1570, XP009096145 ISSN: 0006-4971
- WIRTZ UWE F ET AL: "Pivanex, a histone deacetylase inhibitor, is synergistic with Velcade in inhibiting growth of human non-small cell lung cancer cells." PROCEEDINGS OF THE AMERICAN ASSOCIATION FOR CANCER RESEARCH ANNUAL MEETING, vol. 46, April 2005 (2005-04), pages 1448-1449, XP001536952 & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- ADACHI M ET AL: "Synergistic effect of histone deacetylase inhibitors FK228 and m-carboxycinnamic acid bis-hydroxamide with proteasome inhibitors PSI and PS-341 against gastrointestinal adenocarcinoma cells" CLINICAL CANCER RESEARCH 20040601 US, vol. 10, no. 11, 1 June 2004 (2004-06-01), pages 3853-3862, XP002484085 ISSN: 1078-0432

## Description

The present invention relates to histone deacetylase (HDAC) inhibitors with combined activity on class-I and class-II histone deacetylases. It relates to combinations and compositions comprising them, as well as to their use, as a medicine, for instance as a medicine to inhibit hematopoietic tumors such as lymphomas and leukemias.

The family of HDAC enzymes has been named after their first substrate identified, i.e., the nuclear histone proteins. Histone proteins (H2A, H2B, H3 and H4) form an octamer complex, around which the DNA helix is wrapped in order to establish a condensed chromatin structure. The acetylation status of histones is in dynamic equilibrium governed by histone acetyl transferases (HATs), which acetylate and HDACs which are responsible for the deacetylation of histone tails. Inhibition of the HDAC enzyme promotes the acetylation of the nucleosome histone tails, favoring a more transcriptionally competent chromatin structure, which in turn leads to altered expression of genes involved in cellular processes such as cell proliferation, apoptosis and differentiation. In recent years, a growing number of additional non-histone HDAC substrates have been identified.

Disregulated and constant HDAC recruitment in conjunction with oncogenic transcription factors to the chromatin is observed in specific forms of leukemia and lymphoma, such as acute promyelocytic leukemia (APL), non-Hodgkin's lymphoma and acute myeloid leukemia (AML). Upregulation of HDAC1 at the protein level was observed in prostate cancer cells, as the disease progresses from malignant lesions and well-differentiated androgen-responsive prostate adenocarcinoma towards the phenotypically de-differentiated androgen insensitive prostate cancer. In addition, increased HDAC2 expression is found in the majority of human colon cancer explants which is triggered by the loss of the tumor suppressor adenomatosis polyposis coli (APC).

In agreement with the HDAC/HAT activity equilibrium in cancer, HDAC inhibitors have been shown to induce cell-cycle arrest, terminal differentiation and/or apoptosis in a broad spectrum of human tumor cell lines *in vitro,* to inhibit angiogenesis and to exhibit *in vivo* antitumor activity in human xenograft models in nude mice.

The HDAC family of enzymes are commonly divided into 3 classes: i.e., classes I , II and III. Only Classes I and II have been predominantly implied to mediate the effects of HDAC inhibitors currently in clinical development.

The class-I group HDACs, which consists of HDAC family members 1-3 and 8 have been shown to be crucial for tumor cell proliferation.

Among the wide variety of transcription factors that utilize class-I HDACs to silence specific promotors, the best known example is the class of nuclear hormone receptors, which only bind HDAC3 in absence of their ligand, and thus maintain a state of transcriptional silencing. The complex is dissociated in a ligand-dependent manner, e.g., by retinoids, estrogens, androgens, et cetera, resulting in gene expression and differentiation. Another key example is the HDAC1-dependent silencing of the cyclin-dependent kinase inhibitor p21*^{wafl,cipl}*. The crucial role of p21*^{wafl,cipl}* induction in the antiproliferative effects of HDAC inhibitors was demonstrated by studies showing a 6-fold increase in resistance to the HDAC inhibitor trichostatin A (TSA) in p21*^{wafl,cipl}* deficient cells as compared to the parental HCT-116 cells. In addition, unlike genuine tumor suppressor genes, p21*^{wafl,cipl}* is ubiquitously present in tumor cells, and induced by HDAC inhibitors.

Histones are not the only substrates of the class-I HDACs. For example, HDACs 1-3 deacetylase the tumor suppressor p53, which as a consequence gets ubiquitinated and degraded. Since p53 is a potent tumor suppressor, including cell cycle arrest and apoptosis, maintaining low levels of this protein is key for allowing survival and uncontrolled proliferation of tumor cells.

The class-II HDACs can be divided into 2 subclasses: class-IIa containing HDACs 4, 5, 7, 9 and the HDAC 9 splice variant MITR. Class-IIb comprises HDAC6 and HDAC 10, which both have duplicated HDAC domains. Class-IIa HDACs do not possess intrinsic histone deacetylase activity but regulate gene expression by functioning as the bridging factors since they associate both with class-1 HDAC complexes and with transcription factor/DNA complexes.

HDAC6, a member of class-IIb, has received attention due to its identification as a Hsp90 deacetylase. The HDAC inhibitors LAQ824 and LBH589 have been demonstrated to induce the deacetylation of Hsp90 while trapoxin and sodium butyrate do not. Hsp90 deactylase results in degradation of Hsp90 associated pro-survival and pro-proliferative client proteins. Key examples include Her-2, Bcr-Abl, glucocorticoid receptor, mutant FLT-3, c-Raf and Akt. In addition to Hsp90, HDAC6 also mediates tubulin deacetylation which results in microtubule destabilization under stressed conditions.

The biological role of HDAC6 was further confirmed by the fact that a specific small molecule inhibitor of HDAC6, tubacin, caused α-tubulin hyperacetylation and decreased cell motility without affecting cell cycle progression. Tubacin, which inhibits only the α-tubulin deacetylase domain of HDAC6, causes only a minimal increase in HSP90 acetylation.

In agreement, HDAC6 was found to be key for the estradiol-stimulated cell migration of MCF-7 breast carcinoma cells.

Finally, HDAC6 plays a crucial role in the cellular management of misfolded proteins and clearing these from the cytoplasm.

Due to the large number of cell cycle regulatory proteins regulated by HDACs at the level of either their expression or activity, the antiproliferative effect of HDAC inhibitors cannot be linked to a single mechanism of action. HDAC inhibition holds particular promise in anticancer therapy, where the concerted effects on multiple pathways involved in growth inhibition, differentiation and apoptosis may prove advantageous in the treatment of a heterogeneous pathology such as tumor formation and growth.

Over the years, it has become evident that HDACs do not only play a key role in carcinogenesis, but also in a number of non-malignant differentiation processes. This is most apparent for the class-IIa 4, 5, 7 and 9. For example, HDAC7 has been suggested to play a critical role in the thymic maturation of T-cells, while HDAC4 has been implicated in the regulation of chondrocyte hypertrophy and endochondral bone formation. Most concerns, however, have focused around the role of the class-IIa HDACs in muscle differentiation. HDACs 4, 5, 7 and 9 all suppress the differentiation of myocytes (muscle cells) as a consequence of being transcriptional co-repressors of myocyte enhancer factor 2 (MEF2).

The most common toxicity seen with HDAC inhibitors is myelosuppresion of mild to moderate degree. In addition, nausea/vomiting, fatigue and diarrhea feature as adverse effects in many clinical trials.

EP 1485365 published on 18 September 2003 discloses amongst others the HDAC inhibitor R306465.

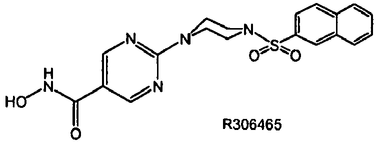

WO 2006/010750 published on 2 February 2006 describes the preparation, formulation and pharmaceutical properties of compounds with the following Markush formula. 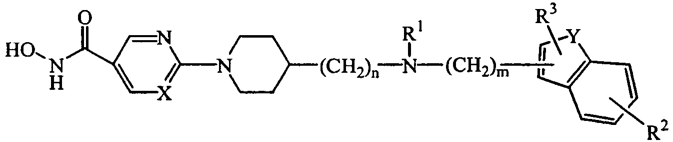 the N-oxide forms, the pharmaceutically acceptable addition salts and the stereochemically isomeric forms thereof,
wherein n, m, R¹, R², R³, X and Y have the meanings as defined in said specification.

The potential for HDAC inhibitor therapy however goes beyond single agent use. The molecular pathways affected by HDAC inhibitors make it a promising candidate for combinatorial studies.

There is a need for inhibitors with combined effects on class-I and class-IIb HDACs that can offer clinical advantages considering efficacy and/or toxicity either alone or in combinations with other therapeutic agents,

Also proteasome inhibition represents an important recently developed strategy in cancer therapy. The proteasome is a multi-enzyme complex present in all cells which play a role in degradation of proteins involved in regulation of the cell cycle. A number of key regulatory proteins, including p53, cyclins and the cyclin-dependent kinase p21*^{wafl,cipl}* are temporally degraded during the cell cycle by the ubiquitin-proteasome pathway. The ordered degradation of these proteins is required for the cell to progress through the cell cycle and to undergo mitosis. Furthermore, the ubiquitin-proteasome pathway is required for transcriptional regulation.

EP788360, EP 1312609, EP 1627880, US 6066730 and US 6083903 discloses peptide boronic ester and acid compounds useful as proteasome inhibitors. One of the compounds N-pyrazinecarbonyl-L-phenylalanine-L-leucineboronic acid (PS-341, now known as bortezomib or Velcade (Millenium)) has antitumor activity in human tumor xenograft models and has received approval for the treatment of patients having relapsed refractory multiple myeloma, and is presently undergoing clinical trials in additional indications, including additional haematological cancers as well as solid tumors. Bortezomib induces cell death by causing a buildup of misfolded and otherwise damaged proteins thereby activating the mitochondrial pathway of apoptosis, for example via Bax- or reactive oxygen species dependent mechanisms.

Bortezomib causes the sequestration of ubiquitin-conjugated proteins into structures termed aggresomes. Aggresomes seem to participate in a cytoprotective response that is activated in response to proteasome inhibition perhaps by shuttling ubiquitylated proteins to lysosomes for degradation.

Bortezomib-induced aggresome formation could be disrupted using the HDAC inhibitor SAHA (suberoylanilide hydroxamic acid). SAHA also demonstrates synergistic effects on apoptosis in vitro and in an orthotopic pancreatic cancer xenograft model in vivo (Cancer Research 2006; 66: (7) 3773-3781).

Another HDAC inhibitor LAQ824 also demonstrate synergistic levels of cell death with bortezomib (Journal of Biological Chemistry 2005; 280: (29) 26729-26734).

The synergistic effect of SAHA and LAQ824 with bortezomib have been related to their HDAC6 inhibitory activity.

There is a further need to increase the inhibitory efficacy of proteasome inhibitors against tumor growth and also to lower dosages of such agents to reduce the potential of adverse toxic side effects to the patient.

At the moment robust data of correlation of the degree of acetylation with tumor response is not available. Quick, simple and easily reproducible methods of quantifying the degree of acetylation of histone and non-histone substrates caused by the below described HDAC inhibitors or combinations comprising said HDAC inhibitors will be crucial to their future.

It is an object of the invention to provide HDAC inhibitors and therapeutic combinations of a proteasome inhibitor and HDAC inhibitors of the type described below which can have robust and characteristic acetylation effects, inhibition of both class-I and class-IIb HDACs, advantageous inhibitory effect against tumor cell growth, and less undesired side effects.

We describe herein a combination of a proteasome inhibitor and a HDAC inhibitor of formula (I) 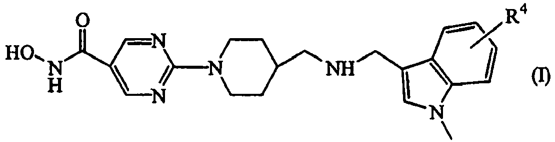 the pharmaceutically acceptable acid or base addition salts and the stereochemically isomeric forms thereof, wherein
R⁴ is selected from hydrogen or halo.

Interesting compounds are those compounds of formula (I) wherein R⁴ is fluor.

More interesting compounds are those compounds of formula (I) wherein R⁴ is in the 4 or the 7 position of the indole.

Preferred compounds of formula (I) are compound No.1a, compound No.30 and compound No.39 corresponding to the numbering as indicated in WO 2006/010750.

| | |
|---|---|
| 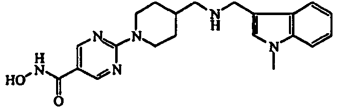 | 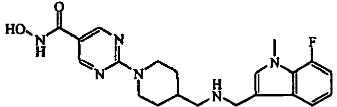 |
| C₂HF₃O₂; Compound 1a | C₂HF₃O₂ (1:1); compound 30 |
| 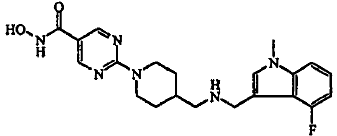 | |
| C₂HF₃O₂ (1:1); compound 39 | |

Other preferred compound of formula (I) are the compounds wherein R⁴ is hydrogen. According to the invention, the HDAC inhibitor of Formula I is compound No. 1a (JNJ26481585) 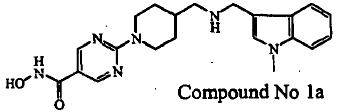 or a pharmaceutically acceptable addition salt thereof, and the proteasome inhibitor is bortezomib.

Lines drawn into the bicyclic ring systems from substituents indicate that the bonds may be attached to any of the suitable ring atoms of the bicyclic ring system.

As used in the foregoing definitions and hereinafter, halo is generic to fluoro, chloro, bromo and iodo.

As used herein, the terms "histone deacetylase" and "HDAC" are intended to refer to any one of a family of enzymes that remove acetyl groups from the ε-amino groups of lysine residues at the N-terminus of a histone. Unless otherwise indicated by context, the term "histone" is meant to refer to any histone protein, including H1, H2A, H2B, H3, H4, and H5, from any species. Human HDAC proteins or gene products, include, but are not limited to, HDAC-1, HDAC-2, HDAC-3,
HDAC-4, HDACs-5, HDAC-6, HDAC-7, HDAC-8, HDAC-9, HDAC-10 and HDAC-11. The histone deacetylase can also be derived from a protozoal or fungal source.

The term "histone deacetylase inhibitor" or "inhibitor of histone deacetylase" is used to identify a compound, which is capable of interacting with a histone deacetylase and inhibiting its activity, more particularly its enzymatic activity. Inhibiting histone deacetylase enzymatic activity means reducing the ability of a histone deacetylase to remove an acetyl group from a histone or another protein substrate. Preferably, such inhibition is specific, i.e. the histone deacetylase inhibitor reduces the ability of a histone deacetylase to remove an acetyl group from a histone or another protein substrate at a concentration that is lower than the concentration of the inhibitor that is required to produce some other, unrelated biological effect.

The term "HDAC inhibitors with combined activity on class-I and class-IIb HDACs" or "inhibition of class-I and class-IIb HDACs" is used to identify compounds which reduce the enzymatic activity of both a class-I HDAC family member (HDAC 1-3 or 8) and a class IIb HDAC family member (HDAC 6 or 10) at a concentration that is lower than the concentration of the inhibitor that is required to produce inhibition of other classes of HDAC enzymes such as e.g. class-IIa or at a concentration that is lower than the concentration of the inhibitor that is required to produce inhibition of some other related biological effect.

As used herein, the terms "proteasome" and "ubiquitin-protesome system (UPS)" are intended to refer to any one of the structures and functions of all components in the UPS which include, but are not limited to:
a) the ubiquitin (Ub) and ubiquitin-like proteins (Ulp); f.e. SUMO, NEDD8, ISG15 and the like,
b) ubiquitin monomers, K48-linked polyubiquitin chains, K63-linked polyubiquitin chains and the like,
c) the E1 ubiquitin-activating enzymes; f.e. E1^{Ub}, E1^{SUMO}, E1^{NEDD8}, E1^{ISG15} and the like,
d) subunits of the E1 ubiquitin-activating enzymes; f.e. APPBP1, UBA3, SAE1, SAE2 and the like,
e) the E2 ubiquitin-conjugating enzymes; f.e. UBC9, UBC12, UBC8 and the like,
f) the E3 ubiquitin ligases; f.e. RING-finger E3s, simple RING-finger E3s, cullin-based RING-finger E3s, RBX1-/RBX2-dependent E3s, HECT-domain E3s, U-box E3s, and the like,
g) the SCF (SKP1-Cullin1-F-box) E3 ubiquitin ligase complex, f.e. SCF^{SKP2}, SCF^{B-TRCP}, SCF^{FBW7} and the like,
h) the cullins, f.e. CUL1, CUL2, CUL3, CUL4,CUL5 and the like,
i) the F-box proteins f.e. SKP2, B-TRCP proteins, FBW proteins and the like,
j) other substrate specific adaptors, f.e. BTB proteins, SOCS-box proteins, DDB1/2, VHL and the like,
k) the proteasome, its components and the like,
l) the metalloisopeptidase RPN11, a subunit of the proteasome lid, that de-ubiquitilates UPS targets prior to their destruction, and the like,
m) the metalloisopeptidase CSN5, a subunit of the COP9-signalosome complex, that is responsible for removing NEDD8 from cullins, and the like,
n) the activation step, by a E1 ubiquitin-activating enzyme, in which the Ub/Ulp first becomes adenylated on its C-terminal glycine residue and then becomes charged as a thiol ester, again at its C-terminus,
o) the transfer of the Ub/Ulp from a E1 ubiquitin-activating enzymes to a E2 ubiquitin-conjugating enzyme,
p) ubiquitin-conjugate recognition,
q) transfer and binding of the substrate-ubiquitin complex to the proteasome,
r) ubiquitin removal, or
s) substrate degradation.

The term "proteasome inhibitor" and "inhibitor of the ubiquitin-proteasome system" is used to identify a compound, which is capable of interacting with one of the normal, altered, hyper-active or overexpressed components in the UPS and inhibiting its activity, more particularly its enzymatic activity. Inhibiting UPS enzymatic activity means reducing the ability of a UPS component to perform its activity. Preferably, such inhibition is specific, i.e. the proteasome inhibitor reduces the activity of a component of the UPS at a concentration that is lower than the concentration of the inhibitor that is required to produce some other, unrelated biological effect. Inhibitors of the activity of a UPS component includes, but are not limited to:
a) inhibitors of Ub or Ulp adenylation by blocking access of Ub/Ulp to the adenylate site or by blocking access of ATP; f.e. imatinib (Gleevec; Novartis) and the like,
b) disruptors of the interaction of the E3 or E3-complex with E2,
c) interrupters of the interaction between the substrate and the substrate interaction domain on the E3 or E3-complex, such as blocking the interaction between p53 (the substrate) and MDM2 (the RING-finger E3) f.e. nutlins (by binding to MDM2), RITA (by binding to the N terminus of p53) and the like,
d) interrupters of the E3 ligase complex,
e) artificially recruiters of substrates to the ubiquitin ligases, f.e. protacs and the like,
f) inhibitors of the proteasome and its components f.e. bortezomib, carfilzomib, NPI-0052, Bsc2118 and the like,
g) inhibitors of the ubiquitin/Ulp removal such as inhibitors of the metalloisopeptidases RPN11 and CSN5, or
h) modifying the polyubiquitin chain f.e. ubistatins and the like.

The pharmaceutically acceptable acid addition salts as mentioned hereinabove are meant to comprise the therapeutically active non-toxic acid addition salt forms which the compounds of formula (I) are able to form. The compounds of formula (I) which have basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, trifluoroacetic, propanoic, hydroxyacetic, lactic, pyruvic, oxalic, malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-amino-salicylic, pamoic and the like acids.

The compounds of formulae (I) which have acidic properties may be converted in their pharmaceutically acceptable base addition salts by treating said acid form with a suitable organic or inorganic base. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The terms acid or base addition salt also comprise the hydrates and the solvent addition forms which the compounds of formulae (I) are able to form. Examples of such forms are e.g. hydrates, alcoholates and the like.

The term stereochemically isomeric forms of compounds of formulae (I) as used hereinbefore, defines all possible compounds made up of the same atoms bonded by the same sequence of bonds but having different three-dimensional structures which are not interchangeable, which the compounds of formulae (I) may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of formulae (I) both in pure form or in admixture with each other are intended to be embraced within the scope of the present invention.

Some of the compounds of formula (I) may also exist in their tautomeric forms. Such forms although not explicitly indicated in the above formula are intended to be included within the scope of the present invention.

Whenever used hereinafter, the term "compounds of formula (I) is meant to include also the pharmaceutically acceptable acid or base addition salts and all stereoisomeric forms.

The proteasome inhibitor for use in accordance with the invention is bortezomib. Bortezomib is commercially available from Millennium under the trade name Velcade and may be prepared for example as described in EP788360, EP1312609,EP1627880, US 6066730 and US 6083903 or by processes analogous thereto.

The present invention also relates to combinations according to the invention for use in medical therapy for example for inhibiting the growth of tumor cells.

The present invention also relates to the use of combinations according to the invention for the preparation of a pharmaceutical composition for inhibiting the growth of tumor cells.

The present invention also relates to a combination for use in inhibiting the growth of tumor cells in a human subject which comprises administering to the subject an effective amount of a combination according to the invention.

This invention further provides a combination for use in inhibiting the abnormal growth of cells, including transformed cells, by administering an effective amount of a combination according to the invention. Abnormal growth of cells refers to cell growth independent of normal regulatory mechanisms (e.g. loss of contact inhibition). This includes the inhibition of tumour growth both directly by causing growth arrest, terminal differentiation and/or apoptosis of cancer cells, and indirectly, by inhibiting migration, invasion and survival of tumor cells or neovascularization of tumors.

This invention also provides a combination for use in inhibiting tumor growth by administering an effective amount of a combination according to the present invention, to a subject, e.g. a mammal (and more particularly a human) in need of such treatment. In particular, this invention provides a combination for use in inhibiting the growth of tumors by the administration of an effective amount of the combination according to the present invention. The present invention is particularly applicable to the treatment of pancreatic cancer, hematopoietic tumors of lymphoid lineage e.g. acute lymphoblastic leukemia, acute myelogenous leukemia, acute promyelocytic leukemia, acute myeloid leukemia, acute monocytic leukemia, lymphoma, chronic B cell leukemia, chronic myeloid leukemia, chronic myeloid leukemia in blast crisis, Burkitt's lymphoma, multiple myeloma, non-small-cell lung cancer, small-cell lung cancer, non-Hodgkin's lymphoma, melanoma, prostate cancer, breast cancer and colon cancer. Examples of other tumors which may be inhibited include, but are not limited to, thyroid follicular cancer, myelodysplastic syndrome (MDS), tumors of mesenchymal origin (e.g. fibrosarcomas and rhabdomyosarcomas), teratocarcinomas, neuroblastomas, gliomas, benign tumor of the skin (e.g. keratoacanthomas), kidney carcinoma, ovary carcinoma, bladder carcinoma and epidermal carcinoma.

This invention also provides a combination for use in the treatment of acute lymphoblastic leukemia, acute myelogenous leukemia, acute promyelocytic leukemia, acute myeloid leukemia, acute monocytic leukemia, lymphoma, chronic B cell leukemia, chronic myeloid leukemia, chronic myeloid leukemia in blast crisis, Burkitt's lymphoma and multiple myeloma by administering an effective amount of a histone deactylase inhibitor of formula (I), to a subject, e.g. a mammal (and more particularly a human) in need of such treatment.

This invention also provides a combination for use in the treatment of drug resistant tumors, such as but not limited to hematopoietic tumors of lymphoid lineage e.g. drug resistant acute lymphoblastic leukemia, drug resistant acute myelogenous leukemia, drug resistant acute promyelocytic leukemia, drug resistant acute myeloid leukemia, drug resistant acute monocytic leukemia, drug resistant lymphoma, drug resistant chronic B cell leukemia, drug resistant chronic myeloid leukemia, drug resistant chronic myeloid leukemia in blast crisis, drug resistant Burkitt's lymphoma and drug resistant multiple myeloma by administering an effective amount of a histone deactylase inhibitor of formula (Ia) or in combination with a bortezomib, to a subject, e.g. a mammal (and more particularly a human) in need of such treatment. The present invention is particularly applicable to the treatment of drug resistant multiple myeloma, more particular to multiple myeloma resistant to proteasome inhibitors, even more particular to the treatment of bortezomib resistant multiple myeloma.

The term "drug resistant multiple myeloma" includes but is not limited to multiple myeloma resistant to one or more drugs selected from the group of thalidomide, dexamethasone, revlimid, doxorubicin, vincristine, cyclophosphamide, pamidronate" melphalan, defibrotide, prednisone, darinaparsin, belinostat, vorinostat, PD 0332991, LBH589, LAQ824, MGCD0103, HuLuc63, AZD 6244, Pazopanib, P276-00, plitidepsin, bendamustine, tanespimycin,enzastaurin, perifosine, ABT-737 or RAD001. The term "drug resistant multiple myeloma" also includes relapsed or refractory multiple myeloma.

With the term "drug resistant" is meant a condition which demonstrates intrinsic resistance or acquired resistance. With "intrinsic resistance" is meant the characteristic expression profile in cancer cells of key genes in relevant pathways, including but not limited to apoptosis, cell progression and DNA repair, which contributes to the more rapid growth ability of cancerous cells when compared to their normal counterparts. With "acquired resistance" is meant a multifactorial phenomenon occurring in tumor formation and progression that can influence the sensitivity of cancer cells to a drug. Acquired resistance may be due to several mechanisms such as but not limited to; alterations in drug-targets, decreased drug accumulation, alteration of intracellular drug distribution, reduced drug-target interaction, increased detoxification response, cell-cycle deregulation, increased damaged-DNA repair, and reduced apoptotic response. Several of said mechanisms can occur simultaneously and/or may interact with each other. Their activation and/or inactivation can be due to genetic or epigenetic events or to the presence of oncoviral proteins. Acquired resistance can occur to individual drugs but can also occur more broadly to many different drugs with different chemical structures and different mechanisms of action. This form of resistance is called multidrug resistance.

The combination according to the invention may be used for other therapeutic purposes,
for example:
a) the sensitisation of tumours to radiotherapy by administering the compound according to the invention before, during or after irradiation of the tumour for treating cancer;
b) treating arthropathies and osteopathological conditions such as rheumatoid arthritis, osteoarthritis, juvenile arthritis, gout, polyarthritis, psoriatic arthritis, ankylosing spondylitis and systemic lupus erythematosus;
c) inhibiting smooth muscle cell proliferation including vascular proliferative disorders, atherosclerosis and restenosis;
d) treating inflammatory conditions and dermal conditions such as ulcerative colitis, Crohn's disease, allergic rhinitis, graft vs. host disease, conjunctivitis, asthma, ARDS, Behcets disease, transplant rejection, uticaria, allergic dermatitis, alopecia areata, scleroderma, exanthema, eczema, dermatomyositis, acne, diabetes, systemic lupus erythematosis, Kawasaki's disease, multiple sclerosis, emphysema, cystic fibrosis and chronic bronchitis;
e) treating endometriosis, uterine fibroids, dysfunctional uterine bleeding and endometrial hyperplasia;
f) treating ocular vascularisation including vasculopathy affecting retinal and choroidal vessels;
g) treating a cardiac dysfunction;
h) inhibiting immunosuppressive conditions such as the treatment of HIV infections;
i) treating renal dysfunction;
j) suppressing endocrine disorders;
k) inhibiting dysfunction of gluconeogenesis;
l) treating a neuropathology for example Parkinson's disease or a neuropathology that results in a cognitive disorder, for example, Alzheimer's disease or polyglutamine related neuronal diseases;
m) treating psychiatric disorders for example schizophrenia, bipolar disorder, depression, anxiety and psychosis;
n) inhibiting a neuromuscular pathology, for example, amylotrophic lateral sclerosis;
o) treating spinal muscular atrophy;
p) treating other pathologic conditions amenable to treatment by potentiating expression of a gene;
q) enhancing gene therapy;
r) inhibiting adipogenesis;
s) treating parasitosis such as malaria.

Hence, the present invention discloses the above described combinations for use as a medicine as well as the use of a HDAC inhibitor of formula (Ia) with combined activity on class-I and class-IIb HDACs, in combination with bortezomib , for the manufacture of a medicament for treating one or more of the above mentioned conditions.

Thus, the present invention discloses the use of a HDAC inhibitor of formula (1a) with combined activity on class-I and class-IIb HDACs, in combination with bortezomib for the manufacture of a medicament for the treatment of acute lymphoblastic leukemia, acute myelogenous leukemia, acute promyelocytic leukemia, acute myeloid leukemia, acute monocytic leukemia, lymphoma, chronic B cell leukemia, chronic myeloid leukemia, chronic myeloid leukemia in blast crisis, Burkitt's lymphoma and multiple myeloma.

The presents invention also discloses the use of a HDAC inhibitor of formula (Ia) with combined activity on class-I and class-IIb HDACs, in combination with bortezomib for the manufacture of a medicament for the treatment of drug resistant tumors, such as but not limited to, hematopoietic tumors of lymphoid lineage e.g. drug resistant acute lymphoblastic leukemia, drug resistant acute myelogenous leukemia, drug resistant acute promyelocytic leukemia, drug resistant acute myeloid leukemia, drug resistant acute monocytic leukemia, drug resistant lymphoma, drug resistant chronic B cell leukemia, drug resistant chronic myeloid leukemia, drug resistant chronic myeloid leukemia in blast crisis, drug resistant Burkitt's lymphoma and drug resistant multiple myeloma.

The present invention further discloses the use of a HDAC inhibitor of formula (Ia) with combined activity on class-I and class-IIb HDACs, in combination wich bortezomib for the manufacture of a medicament for the treatment of drug resistant multiple myeloma, more in particular of multiple myeloma resistant to proteasome inhibitors, even more in particular of bortezomib resistant multiple myeloma.

Bortezomib and the HDAC inhibitor of formula (Ia) may be administered simultaneously (e.g. in separate or unitary compositions) or sequentially in either order. In the latter case, the two compounds will be administered within a period and in an amount and manner that is sufficient to ensure that an advantageous or synergistic effect is achieved.

The present invention further relates to a product containing as first active ingredient a HDAC inhibitor of formula (Ia) (I) and bortezomib, as a combined preparation for simultaneous, separate or sequential use in the treatment of patients suffering from cancer.

Those skilled in the art could easily determine the effective amount from the test results presented hereinafter. In general it is contemplated that a therapeutically effective amount of a compound of formula (I) and of a proteasome inhibitor would be from 0.005 mg/kg to 100 mg/kg body weight, and in particular from 0.005 mg/kg to 10 mg/kg body weight. It may be appropriate to administer the required dose as two, three, four or more sub-doses at appropriate intervals throughout the day. Said sub-doses may be formulated as unit dosage forms, for example, containing 0.5 to 500 mg, and in particular 10 mg to 500 mg of active ingredient per unit dosage form.

In view of their useful pharmacological properties, the components of the combinations according to the invention may be formulated into various pharmaceutical forms for administration purposes. The components may be formulated separately in individual pharmaceutical compositions or in a unitary pharmaceutical composition containing both components.

The present invention therefore also relates to a pharmaceutical composition comprising bortezomib and a HDAC inhibitor of formula (Ia) together with one or more pharmaceutical carriers. To prepare pharmaceutical compositions for use in accordance with the invention, an effective amount of a particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

It may be appropriate to administer the required dose of each component of the combination as two, three, four or more sub-doses at appropriate intervals throughout the course of treatment. The sub-doses may be formulated as unit dosage forms, for example, in each case containing independently 0.01 to 500 mg, for example 0.1 to 200 mg and in particular 1 to 100 mg of each active ingredient per unit dosage form.

The term "the induction of acetylation of histones or other proteins" means the induction of the acetylation status of HDAC substrates such as but not limited to histones, e.g. histone 3, histone 4 and the like; tubulin, e.g. alpha-tubulin and the like; heat shock proteins, e.g. Hsp 90 and the like.

The term "the induction of proteins functionally regulated by said acetylation" means secondary effects such as but not limited to induction of Hsp70, induction of p21 and the like.

We also describe a method for the characterisation of HDAC inhibitor of formula (I) either alone or in combination with a proteasome inhibitor comprising the determination in a sample, of the amount of induction of acetylation of histones or other proteins, or of the induction of proteins functionally regulated by said acetylation. More in particular, the invention relates to a method for the characterisation of a HDAC inhibitor of formula (I) either alone or in combination with a proteasome inhibitor, comprising the determination in a sample of the amount of
a) induction of acetylation of histone 3, induction of acetylation of histone 4, or induction of p21 and
b) induction of acetylation of alpha-tubulin, induction of acetylation of Hsp 90, or induction of Hsp 70.

Most particular the invention relates to the above method, wherein the concentration needed to obtain induction under a) is in the same range as the concentration to obtain induction under b).

The determination in a sample of the amount of induction of acetylation of histones or other proteins or of the induction of proteins functionally regulated by said acetylation may encompass the identification of patients that respond to a treatment and thus may have a beneficial effect for the treatment of human cancer.

The determination in a sample of the amount of induction of acetylation of histones or other proteins or of the induction of proteins functionally regulated by said acetylation may encompass monitoring efficacy of a treatment in patients and thus may have a beneficial effect for the treatment of human cancer.

The determination in a sample of the amount of induction of acetylation of histones or other proteins or of the induction of proteins functionally regulated by said acetylation may encompass predicting therapeutic responses to a treatment and thus may have a beneficial effect for the treatment of human cancer.

Hence we describe also the use of a HDAC inhibitor of formula (Ia) with combined activity on class-I and class-IIb HDACs, in combination with bortezomib, wherein the induction of hyperacetylation of histones or other proteins or the induction of proteins functionally regulated by said acetylation has a beneficial effect for the treatment of human cancer.

The sample may be derived from cells which have been treated with said combination. The sample may also be derived from tissue affected by a disorder and/or from individuals treated with a combination of bortezomib and a HDAC inhibitor of formula (Ia).

The cells may be culture cells which have been contacted with said combination. Said combination can be added to the growth medium of the cells.

The cells may also be derived from a tissue and/or from an individual that was treated with said combination.

Preferably, the method of characterization comprises only steps which are carried out *in vitro.* Therefore, according to this embodiment the step of obtaining the tissue material from the human or animal body is not encompassed by the present invention.

The cells are usually processed to be in a condition which is suitable for the method employed, for determining the induction of acetylation of histones or other proteins or the induction of proteins functionally regulated by said acetylation. Processing may include homogenization, extraction, fixation, washing and/or permeabilisation. The way of processing largely depends on the method used for the determination of the induction of acetylation of histones or other proteins or the induction of proteins functionally regulated by said acetylation. The sample may be derived from a biopsy of the patent. The biopsy may be further treated to yield a sample which is in a condition suitable for the method used for determining the induction of acetylation of histones or other proteins or the induction of proteins functionally regulated by said acetylation.

The amount of acetylation of proteins or the amount of induced protein may be determined by use of an antibody.

As used herein, the term "antibody" designates an immunoglobulin or a derivative thereof having the same binding specificity. The antibody used according to the invention may be a monoclonal antibody or an antibody derived from or comprised in a polyclonal antiserum. The term "antibody" further means derivatives such as Fab, F(ab')2, Fv or scFv fragments. The antibody or the derivative thereof may be of natural origin or may be (semi)synthetically produced.

Western blotting may be used which is generally known in the art. The cellular material or tissue may be homogenized and treated with denaturing and/or reducing agents to obtain the samples. The sample may be loaded on a polyacrylamide gel to separate the proteins followed by transfer to a membrane or directly be spotted on a solid phase. The antibody is then contacted with the sample. After one or more washing steps the bound antibody is detected using techniques which are known in the art.

Immunohistochemistry may be used after fixation and permeabilisation of tissue material, e.g. slices of solid tumors, the antibody is then incubated with the sample, and following one or more washing steps the bound antibody is detected.

The amount of the induction of acetylation of histones or other proteins or the induction of proteins functionally regulated by said acetylation may be determined by ELISA. A variety of formats of the ELISA can be envisaged. In one format, the antibody is immobilized on a solid phase such as a microtiter plate, followed by blocking of aspecific binding sites and incubation with the sample. In another format, the sample is first contacted with the solid phase to immobilize the acetylated and/or induced proteins contained in the sample. After blocking and optionally washing, the antibody is contacted with the immobilized sample.

The amount of the induction of acetylation of histones or other proteins or the induction of proteins functionally regulated by said acetylation may be determined by flow cytometry. Cells, e.g. cell culture cells or blood cells or cells from bone marrow, are fixed and permeabilised to allow the antibody to reach the acetylated and/or induced proteins. After optional washing and blocking steps the antibody is contacted with the cells. Flow cytometry is then performed in accordance with procedures known in the art in order to determine cells having antibody bound to the acetylated and/or induced proteins.

To determine whether a HDAC inhibitor or a combination of a proteasome inhibitor and a HDAC inhibitor of formula (I) has his activity, one may determine the amount of acetylation of a protein or induction of a protein in a reference sample wherein the reference sample is derived from cells which have not been treated with said HDAC inhibitor or said combination. The determination of the amount of acetylation of proteins and/or the amount of induced protein in the sample and the reference sample may be performed in parallel. In the case of cell culture cells, two cellular compositions are provided, one of which is treated with said HDAC inhibitor or said combination whereas the other is left untreated. Subsequently both compositions are further processed and the respective amounts of acetylation of proteins and/or the amount of induced protein are determined. Alternatively, to determine whether a HDAC inhibitor or a combination of a proteasome inhibitor and a HDAC inhibitor of formula (I) has his activity, one may determine inhibition of cell proliferation.

In the case of patients, the sample is derived from a patient which has been treated with the HDAC inhibitor of formula (I) or the combination of a proteasome inhibitor and a HDAC inhibor of formula (I). The reference sample is derived from another patient suffering from the same disorder who has not been treated with said HDAC inhibitor or said combination or from a healthy individual. The tissue from which the reference sample is derived corresponds to the tissue from which the sample is derived. For example, if the sample is derived from tumor tissue from a breast cancer patient the reference sample is also derived from tumor tissue from a breast cancer patient or from breast tissue from a healthy individual. It may also be envisaged that the sample and the reference sample are derived from the same individual. In this case, the tissue, from which the reference sample is derived was obtained from the individual prior to or after treatment of the individual with said HDAC inhibitor or said combination. Preferably, the tissue is obtained prior to the treatment to exclude possible after-effects of the inhibitor treatment after discontinuation of the treatment.

### Experimental part

### A. Pharmacological example

For the Cellular activity of the compounds of formula (I) which was determined on A2780 tumour cells using a colorimetric assay for cell toxicity or survival (Mosmann Tim, Journal of Immunological Methods 65: 55-63, 1983), reference is made to the experimental part of WO 2006/010750

The antiproliferative effects of HDAC inhibitors has been linked to the inhibition of class 1 HDACs, which consists of HDAC family members 1-3 and 8. The activity of JNJ 26481585 on HDAC 1 immuno-precipitated from A2780 cells and its potency when compared with R306465, SAHA, LBH-589 and LAQ-824 can be found in example A.1..The activity of JNJ 26481585 on HDAC 8 human recombinant enzyme and its potency when compared with R306465, SAHA, LBH-589 and LAQ-824 can be found in example A.2..

It was further investigated whether R306465 modulates the acetylation status of HDAC 1 substrates Histone 3 (H3) and Histone 4 (H4). Also the induction of cyclin dependent kinase inhibitor p21^{wafl,cipl} in A2780 ovarian carcinoma cells was investigated. P21^{wafl,cipl} is repressed as a consequence of histone acetylation, and plays a key role in the induction of cell cycle arrest in response to HDAC inhibitors (see example A.3.).

In order to assess the inhibition of HDAC 6, and the relative potency of the compounds for HDAC 1 versus HDAC 6, the acetylation of its substrate tubulin, and the induction of Hsp 70, which is the consequence of Hsp 90 acetylation, was monitored (see example A.4.)..

### Example A: class-I specificity and acetylation effects of the compounds of formula (I)

### Example A.1: inhibition of HDAC 1 enzyme immuno-precipitated from A2780 cells

For HDAC1 activity assays, HDAC1 was immunoprecipitated from A2780 cell lysates and incubated with a concentration curve of the indicated HDAC inhibitor, and with a [³H]acetyl-labeled fragment of H4 peptide (50.000 cpm) [biotin-(6-aminohexanoic)Gly-Ala-(acetyl[³H]Lys-Arg-His-Arg-Lys-Val-NH₂](Amersham Pharmacia Biotech, Piscataway, NJ). HDAC activity was assessed measuring release of free acetyl groups. Results are expressed as average IC₅₀ values ± SD for three independent experiments.

| | HDAC 1 inhibition IC₅₀ in nM |
|---|---|
| JNJ 26481585 | 0.16 ± 0.02 |
| R306465 | 3.31 ± 0.78 |
| SAHA | 73 ± 26 |
| LAQ-824 | 0.29 ± 0.05 |
| LBH-589 | 0.23 ± 0.06 |

### Example A.2: inhibition of HDAC 8 human recombinant enzyme

For the inhibition of human recombinant HDAC 8, the HDAC 8 Colorimetric/Flourimetric Activity Assay/Drug Discovery Kit (Biomol; Cat. nr. AK-508) was used. Results are expressed as average IC₅₀ values (nM) ± SD for three independent experiments. Assays were performed in duplicate and the standard error of the IC₅₀ was calculated using Graphpad Prism (Graphpad Software).

| | HDAC 8 inhibition IC₅₀ in nM |
|---|---|
| JNJ 26481585 | 34 ± 41 |
| R306465 | 23 ± 17 |
| SAHA | 370 ± 314 |
| LAQ-824 | 37 ± 23 |
| LBH-589 | 283 ± 29 |

### Example A.3: Acetylation of cellular HDAC 1 substrates and induction of p21^{wafl,cipl}

Human A2780 ovarian carcinoma cells were incubated with 0, 1, 3, 10, 30, 100, 300, 1000 and 3000 nM of the compounds for 24h.

Total cell lysates were prepared and analysed by SDS-PAGE. Levels of acetylated H3 and H4 histones, total level of H3 proteins and levels p21^{wafl,cipl} protein were detected using rabbit polyclonal and mouse monoclonal antibodies, followed by enhanced chemoluminescence (ECL) detection.

Levels of acetylated H3 and H4 were detected with antibodies from Upstate Biotechnology (Cat. nr. 06-299 and 06-866), total level of H3 proteins was detected with antibodies from Abcam (Cat. nr. ab1791) and level of p21^{wafl,cipl} protein was detected with antibodies from Transduction Laboratories (Cat. nr. C24420). Appropriate dilutions of antibodies were incubated for either 1-2 h at room temperature or overnight at 4°C. In order to control for equal loading, blots were stripped and re-probed with mouse monoclonal anti-actin IgM (Ab-1, Oncogene Research Products). in order to control the efficiency of extraction of nuclear proteins blots were stripped and re-probed with and with anti-lamin B1 (Zymed; Cat. nr. 33.2000). Protein-antibody complexes were then visualized by chemiluminescence (Pierce Chemical Co) or fluorescence (Odyssey) according to the manufacturer's instructions. The experiments were performed three times.

| | concentration (nM) when induction of acetylation of histone H3 and H4 and induction of p21^{wafl,cipl} is observed |
|---|---|
| JNJ 26481585 | 10 |
| R306465 | 100 |
| SAHA | 3000 |
| LAQ-824 | 10 |
| LBH-589 | 10 |

### Example A.4. Acetylation of Tubulin and the induction of Hsp 70

Human A2780 ovarian carcinoma cells were incubated with 0, 1, 3, 10, 30, 100, 300, 1000 and 3000 nM of the compounds for 24h.

Total cell lysates were prepared and analysed by SDS-PAGE. Levels of total and acetylated tubulin were detected using antibodies from Sigma: clones DM1A (Cat. nr. T9026) and 6-111B (Cat. nr. T6793). Hsp 70 protein was detected with an antibody from Stressgen (Cat. nr. SPA-810), followed by ECL detection. Appropriate dilutions of antibodies were incubated for either 1-2 h at room temperature or overnight at 4°C. In order to control for equal loading, blots were stripped and re-probed with mouse monoclonal anti-actin IgM (Ab-1, Oncogene Research Products). In order to control the efficiency of extraction of nuclear proteins blots were stripped and re-probed with and with anti-lamin B1(Zymed; Cat. nr. 33.2000). Protein-antibody complexes were then visualized by chemiluminescence (Pierce Chemical Co) or fluorescence (Odyssey) according to the manufacturer's instructions. The experiments were performed three times.

| | concentration (nM) when start of induction of acetylation of tubulin and induction of Hsp 70 is observed |
|---|---|
| JNJ 26481585 | 30 |
| R306465 | 1000 |
| SAHA | 100 |
| LAQ-824 | 30 |
| LBH-589 | 30 |

### Example B: Inhibition of human hematological tumor cell proliferation

Evaluation of anti-proliferative activity of JNJ 26481585 in a pannel of human hematological tumor cell lines was outsourced at Oncodesign (Dijon, France). Tumor cells were grown as cell suspension in the corresponding appropriate culture medium at 37°C in a humidified 5% CO₂ incubator. Mycoplasma-free tumor cells were seeded in 96-well flat-bottom microtitration plates and incubated at 37°C for 24 hr in culture medium containing 10% FCS. Tumor cells were then exposed to vehicle (control) or increasing concentrations of JNJ 26481585 (5 different concentrations*), Bortezomib (5 different concentrations*), or combination of both drugs at various ratio. Cells were then incubated for an additional 72 hr. The cytotoxic activity of the compound(s) was revealed by standard MTS assay by measurement of absorbency at 490 nm. The compound interactions (synergy, additivity or antagonism) was calculated by multiple drug effect analysis and was performed by the median equation principle according to the methodology described by Chou & Talalay [CHOU et al. (1984) Adv. Enzyme Regul. 22: 27-55; CHOU et al. (1991) in Encyclopaedia of human Biology. Academic Press. 2: 371-379; CHOU et al. (1991) in Synergism and antagonism in chemotherapy. Academic Press: 61-102; CHOU et al. (1994) J. Natl. Cancer Inst. 86: 1517-1524]
*: based on pre-determination of anti-proliferative activity of each drug used as single agent, concentrations were chosen not to exceed 50% inhibition in each of the selected cell lines.

### Example B.1.: Inhibition of human hematological tumor cell proliferation by JNJ 26481585.

**Table F.1: Results are expressed as the mean IC₄₀ value (i.e., concentration, expressed in nM, required to reach 40% inhibition of cell proliferation) ± SD, determined from 3 independent reliable experiments.**

| Cell line | Type | Mean | *SD* |
|---|---|---|---|
| CCRF-CEM | Acute lymphoblastic leukemia | 11.93 | *7.18* |
| Jurkat clone E6-1 | Acute lymphoblastic leukemia | 9.22 | *1.78* |
| KG-1 | Acute myelogenous leukemia | 13.39 | *10.48* |
| MOLT-4 | Acute lymphoblastic leukemia | 42.96 | *56.25* |
| SUP-B15 | Acute lymphoblastic leukemia | 1.13 | |
| HL-60 | Acute promyelocytic leukemia | 24.28 | *10.72* |
| OCI-AML2 | Acute myeloid leukemia | 19.56 | *13.93* |
| THP-1 | Acute monocytic leukemia | 50.65 | *22.84* |
| EHEB | Chronic B cell leukemia | 165.81 | *128.15* |
| BV-173 | Chronic B cell leukemia | 4.54 | *4.28* |
| K-562 | Chronic myeloid leukemia | 15.91 | *8.29* |
| KCL-22 | Chronic myeloid leukemia | 7.71 | *5.14* |
| LAMA-84 | Chronic myeloid leukemia in blast crisis | 30.40 | *19.93* |
| U-937 | Lymphoma | 23.67 | *10.14* |
| Daudi | Burkitt's lymphoma | 9.08 | *1.27* |
| Namalwa | Burkitt's lymphoma | 4.65 | *4.32* |
| Raji | Burkitt's lymphoma | 26.34 | *16.11* |
| Ramos | Burkitt's lymphoma | 4.66 | *2.82* |
| ARH-77 | Myeloma | 40.42 | *24.32* |
| RPMI 8226 | Myeloma | 6.41 | *5.44* |

### Example B.2.: Inhibition of human hematological tumor cell proliferation by Bortezomib.

**Table F.2: Results are expressed as the mean IC₄₀ value (i.e., concentration, expressed in nM, required to reach 40% inhibition of cell proliferation) ± SD, determined from 3 independent reliable experiments.**

| Cell line | Type | Mean | *SD* |
|---|---|---|---|
| CCRF-CEM | Acute lymphoblastic leukemia | 4.40 | *0.84* |
| Jurkat clone E6-1 | Acute lymphoblastic leukemia | 5.63 | *2.68* |
| KG-1 | Acute myelogenous leukemia | 3.36 | *0.83* |
| MOLT-4 | Acute lymphoblastic leukemia | 12.14 | *12.91* |
| SUP-B15 | Acute lymphoblastic leukemia | 2.40 | |
| HL-60 | Acute promyelocytic leukemia | 13.38 | *1.99* |
| OCI-AML2 | Acute myeloid leukemia | 11.64 | *11.61* |
| THP-1 | Acute monocytic leukemia | 5.83 | *0.94* |
| EHEB | Chronic B cell leukemia | 6.02 | *0.34* |
| BV-173 | Chronic B cell leukemia | 2.77 | *0.20* |
| K-562 | Chronic myeloid leukemia | 12.83 | *4.11* |
| KCL-22 | Chronic myeloid leukemia | 1.74 | *1.56* |
| LAMA-84 | Chronic myeloid leukemia in blast crisis | 2.61 | *0.46* |
| U-937 | Lymphoma | 5.68 | *1.07* |
| Daudi | Burkitt's lymphoma | 2.68 | *0.54* |
| Namalwa | Burkitt's lymphoma | 4.48 | *1.00* |
| Raji | Burkitt's lymphoma | 5.20 | *0.69* |
| Ramos | Burkitt's lymphoma | 1.83 | *0.10* |
| ARH-77 | Myeloma | 7.21 | *2.22* |
| RPMI 8226 | Myeloma | 4.23 | *0.99* |

### Example B.3: Inhibition of human hematological tumor cell proliferation by JNJ 26481585 in combination with Bortezomib.

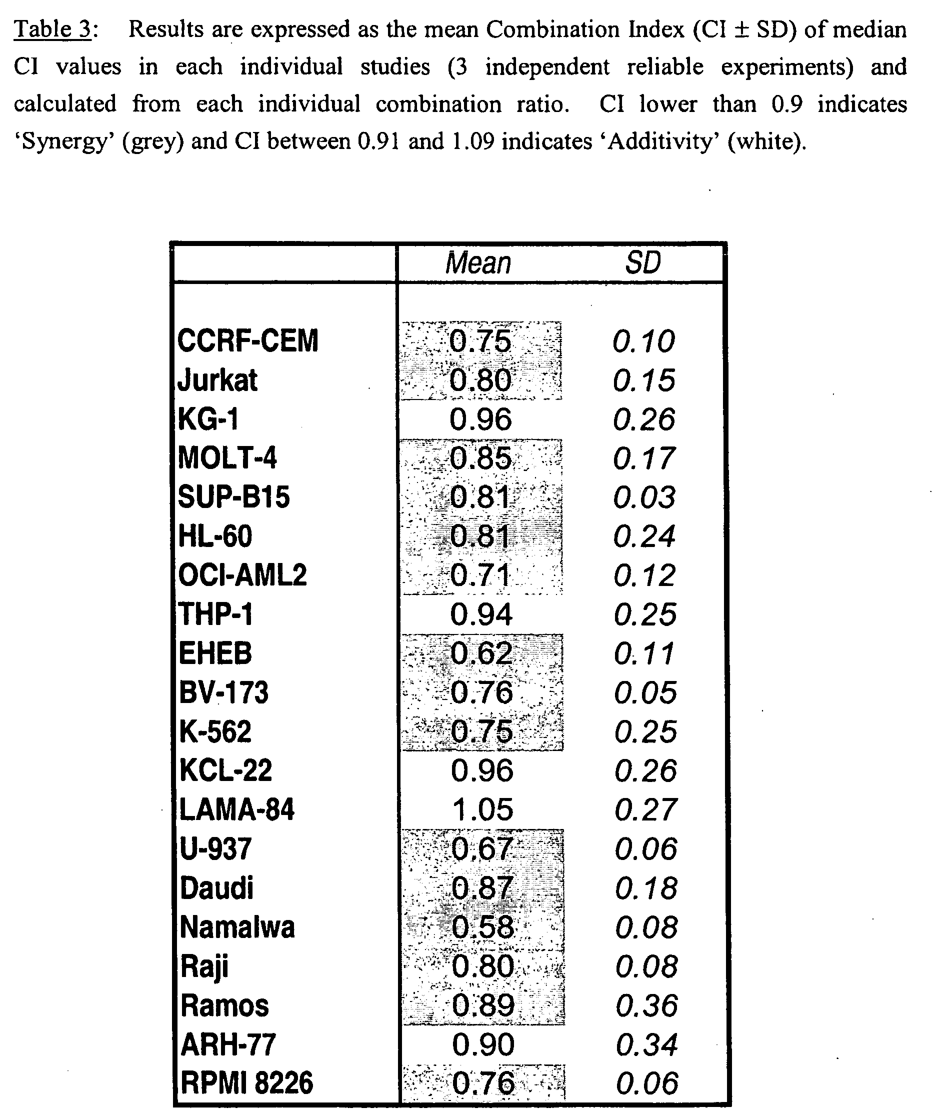

## Claims

1. A combination of a proteasome inhibitor, wherein said proteasome inhibitor is bortezomib, and a histone deacetylase inhibitor wherein said histone deacetylase inhibitor is compound No. 1a (JNJ26481585) 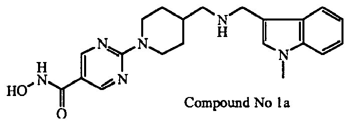 the pharmaceutically acceptable acid or base addition salts and the stereochemically isomeric forms thereof.

2. A combination as claimed in claim 1 in the form of a pharmaceutical composition comprising bortezomib and compound No. 1a together with one or more pharmaceutical carriers.

3. A combination as claimed in claim 2 for simultaneous, separate or sequential use.

4. A combination as claimed in any of claims 1 to 3 for use in medical therapy.

5. Use of a combination as claimed in any of claims 1 or 2 for the manufacture of a medicament for inhibiting the growth of tumor cells.

6. Use of a histone deacetylase inhibitor in combination with a proteasome inhibitor, for the manufacture of a medicament for the treatment of acute lymphoblastic leukemia, acute myelogenous leukemia, acute promyelocytic leukemia, acute myeloid leukemia, acute monocytic leukemia, lymphoma, chronic B cell leukemia, chronic myeloid leukemia, chronic myeloid leukemia in blast crisis, Burkitt's lymphoma and multiple myeloma wherein said histone deactylase inhibitor is compound No. 1a 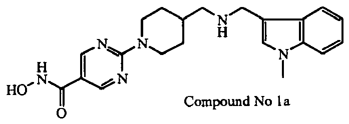 the pharmaceutically acceptable acid or base addition salts and the stereochemically isomeric forms thereof,
and wherein said proteasome inhibitor is bortezomib.

7. Use as claimed in claim 6 wherein said medicament is for treatment of drug resistant acute lymphoblastic leukemia, drug resistant acute myelogenous leukemia, drug resistant acute promyelocytic leukemia, drug resistant acute myeloid leukemia, drug resistant acute monocytic leukemia, drug resistant lymphoma, drug resistant chronic B cell leukemia, drug resistant chronic myeloid leukemia, drug resistant chronic myeloid leukemia in blast crisis, drug resistant Burkitt's lymphoma and drug resistant multiple myeloma.

8. Use as claimed in claims 6 and 7 wherein said medicament is for treatment of bortezomib resistant multiple myeloma.

## Patentansprüche

1. Kombination aus einem Proteasomhemmer, wobei es sich bei dem Proteasomhemmer um Bortezomib handelt, und einem Histon-Deacetylase-Hemmer, wobei es sich bei dem Histon-Deacetylase-Hemmer um die Verbindung Nr. 1a (JNJ26481585) 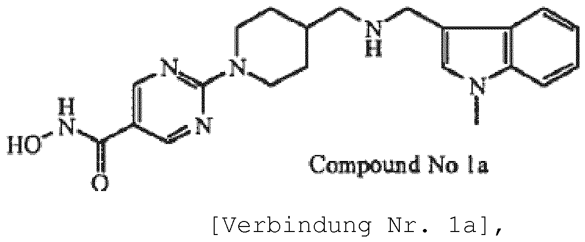 die pharmazeutisch unbedenklichen Säure- oder Basenadditionssalze und die stereochemisch isomeren Formen davon handelt.

2. Verbindung nach Anspruch 1 in Form einer pharmazeutischen Zusammensetzung, die Bortezomib und Verbindung Nr. 1a gemeinsam mit einem oder mehreren pharmazeutischen Trägern enthält.

3. Kombination nach Anspruch 2 für die gleichzeitige, getrennte oder aufeinanderfolgende Verwendung.

4. Kombination nach einem der Ansprüche 1 bis 3 für die Verwendung in der medizinischen Therapie.

5. Verwendung einer Kombination nach einem der Ansprüche 1 oder 2 für die Herstellung eines Arzneimittels zum Hemmen des Wachstums von Tumorzellen.

6. Verwendung eines Histon-Deacetylase-Hemmers in Kombination mit einem Proteasomhemmer für die Herstellung eines Arzneimittels für die Behandlung von akuter lymphoblastischer Leukämie, akuter myelogener Leukämie, akuter promyelozytärer Leukämie, akuter myeloischer Leukämie, akuter Monozytenleukämie, Lymphom, chronischer B-Zellen-Leukämie, chronischer myeloischer Leukämie, chronischer myeloischer Leukämie in der Blastenkrise, Burkitt-Lymphom und multiples Myelom, wobei es sich bei dem Histon-Deacetylase-Hemmer um Verbindung Nr. 1a 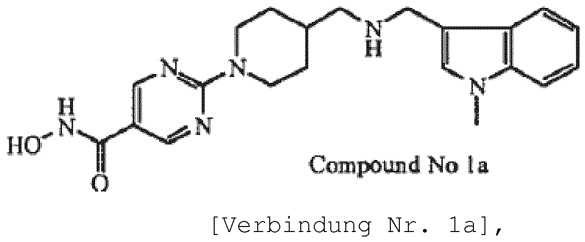 die pharmazeutisch unbedenklichen Säure- oder Basenadditionssalze und die stereochemisch isomeren Formen davon handelt
und wobei es sich bei dem Proteasomhemmer um Bortezomib handelt.

7. Verwendung nach Anspruch 6, wobei das Medikament der Behandlung von arzneimittelresistenter akuter lymphoblastischer Leukämie, arzneimittelresistenter akuter myelogener Leukämie, arzneimittelresistenter akuter promyelozytärer Leukämie, arzneimittelresistenter akuter myeloischer Leukämie, arzneimittelresistenter akuter Monozytenleukämie, arzneimittelresistentem Lymphom, arzneimittelresistenter chronischer B-Zellen-Leukämie, arzneimittelresistenter chronischer myeloischer Leukämie, arzneimittelresistenter chronischer myeloischer Leukämie in der Blastenkrise, arzneimittelresistentem Burkitt-Lymphom und arzneimittelresistentem multiplem Myelom dient.

8. Verwendung nach den Ansprüchen 6 und 7, wobei das Arzneimittel der Behandlung von bortezomibresistentem multiplem Myelom dient.

## Revendications

1. Combinaison d'un inhibiteur de protéasomes, ledit inhibiteur de protéasomes étant le bortézomib, et d'un inhibiteur de l'histone désacétylase, ledit inhibiteur de l'histone désacétylase étant le composé n°1a (JNJ26481585) 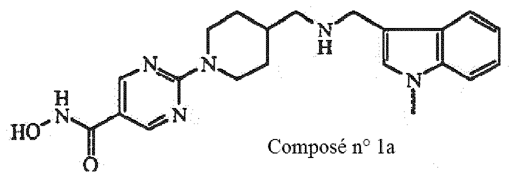 les sels d'addition d' acides ou de bases pharmaceutiquement acceptables et les formes stéréochimiquement isomères de celui-ci.

2. Combinaison selon la revendication 1, sous forme d'une composition pharmaceutique comprenant le bortézomib et le composé n° 1a conjointement avec un ou plusieurs supports pharmaceutiques.

3. Combinaison selon la revendication 2, destinée à une utilisation simultanée, séparée ou séquentielle.

4. Combinaison selon l'une quelconque des revendications 1 à 3, destinée à être utilisée en thérapie médicale.

5. Utilisation d'une combinaison selon l'une ou l'autre des revendications 1 et 2, pour la fabrication d'un médicament destiné à inhiber la croissance des cellules tumorales.

6. Utilisation d'un inhibiteur de l'histone désacétylase en combinaison avec un inhibiteur de protéasomes, pour la fabrication d'un médicament destiné au traitement de la leucémie lymphoblastique aiguë, de la leucémie myélogène aiguë, de la leucémie promyélocytaire aiguë, de la leucémie myéloïde aiguë, de la leucémie monocytaire aiguë, d'un lymphome, de la leucémie chronique à cellules B, de la leucémie myéloïde chronique, de la leucémie myéloïde chronique en crise blastique, du lymphome de Burkitt et du myélome multiple, ledit inhibiteur de l'histone désacétylase étant le composé n° 1a 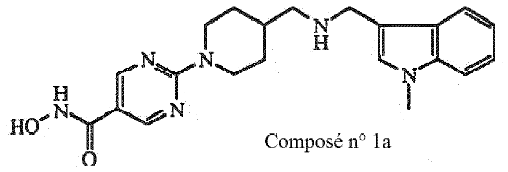 les sels d'addition d'acides ou de bases pharmaceutiquement acceptables et les formes stéréochimiquement isomères de celui-ci,
et ledit inhibiteur de protéasomes étant le bortézomib.

7. Utilisation selon la revendication 6, **caractérisée en ce que** ledit médicament est destiné au traitement de la leucémie lymphoblastique aiguë résistante aux médicaments, de la leucémie myélogène aiguë résistante aux médicaments, de la leucémie promyélocytaire aiguë résistante aux médicaments, de la leucémie myéloïde aiguë résistante aux médicaments, de la leucémie monocytaire aiguë résistante aux médicaments, d'un lymphome résistant aux médicaments, de la leucémie chronique à cellules B résistante aux médicaments, de la leucémie myéloïde chronique résistante aux médicaments, de la leucémie myéloïde chronique en crise blastique résistante aux médicaments, du lymphome de Burkitt résistant aux médicaments et du myélome multiple résistant aux médicaments.

8. Utilisation selon les revendications 6 et 7, **caractérisée en ce que** ledit médicament est destiné au traitement du myélome multiple résistant au bortézomib.
